# EUROPEAN PATENT SPECIFICATION

(11) **EP 1 784 176 B1**
(45) Date of publication and mention of the grant of the patent: **14.04.2010**
(21) Application number: 05755054.3
(22) Date of filing: 23.06.2005
(51) Int. Cl.: A61K 31/40, A61P 31/04, A61K 38/40

(54) **COMPOSITION COMPRISING LACTIC ACID AND LACTOFERRIN**
ZUSAMMENSETZUNG AUS MILCHSÄURE UND LACTOFERRIN
COMPOSITION COMPRENANT DE L'ACIDE LACTIQUE ET DE LA LACTOFERRINE

(30) Priority: 23.06.2004 SE 0401631
(43) Date of publication of application: 16.05.2007
(73) Proprietor: Nestor Medical AB, 646 23 Gnesta (SE)
(72) Inventor: MATTSBY-BALTZER, Inger, S-412 70 Göteborg (SE); ANDERSCH, Björn, S-429 43 Göteborg (SE)
(74) Representative: Andersson, Inga-Lill
(86) International application number: PCT/SE2005/000989
(87) International publication number: WO 2006/001766

(56) References cited:
- EP-A1- 0 257 007
- EP-A1- 0 629 347
- EP-A1- 1 040 766
- EP-A1- 1 083 964
- WO-A1-02/40013
- WO-A1-98/06425

## Description

### Technical field

The present invention relates to a composition comprising lactic acid in combination with lactoferrin for use in the treatment and/or prophylaxis of conditions in the urogenital tract caused by C. albicans.

It also relates to the use of such composition for the preparation of a medicament for the treatment and/or prophylaxis of conditions in the urogenital tract caused by C. albicans as well as to a method for the treatment and/or prophylaxis of conditions in the urogenital tract caused by C. albicans.

### Background of the invention

Acidity is believed to be one of the protective mechanisms of the vagina. This acidity has been associated with a decreased risk of infections such as chlamydia, genital mycoplasma, trichomoniasis, urinary tract infection and a decreased carriage of bacteria in the introitus (Hanna, N et al 1985, 1975, Stamey TA and Kaufman MF 1975, Stamey TA and Timothy MM 1975).

In contrast, imbalance of the vaginal microbiota, bacterial vaginosis (BV), has been associated with premature birth, increased risk of sexually transmitted diseases, increased risk of HIV infection, and pelvic inflammatory disease (Schwebke, JR, 2003, Obstet Gynecol Clin North Am. 30:685-94; Eschenbach, DA, 1993, Am J Obstet Gynecol, Vol 169:441-5; Taha et al, Aids, 1998, Vol 12:1699-706, Hillier, SL, et al, 1996, Am J Obstet Gynecol, Vol 175: 435-41).

During the perinatal period and from menarche to menopause the vagina is most acidic. The vagina is believed to be acidified by the anaerobic metabolism of vaginal glycogen to acidic products, predominantly acetic and lactic acids. Whether this metabolism is performed by vaginal bacteria and/or epithelial cells is still under debate (see review Pybus and Onderdonk 1999).

A favourable environment for the lactate producing microbiota is generally present, and results in a pH of approximately 4 in the mucousal secretions. However, the environment can be disturbed by factors such as variations in the oestrogen concentration during certain periods of the menstrual cycle and during the menopause, and also by an increasing occurrence of secretions of various types, for example from protracted menstrual haemorrhages, premenstrual bloody discharges, mid-cycle bleeding and ejaculate. Foreign bodies in the vagina, for example coils, pessaries and those inserted, for example, in connection with antibiotic treatment and on washing can also lead to a disruption of the healthy microbiota which is such that the pH rises. When the pH rises above 4.5 there is an increased tendency towards growth of anaerobic bacteria.

Disruptions of the normal microbiota in the vagina without inflammatory reactions in the mucous membrane are referred to as BV and lead to offensive discharges and pH's over 4.5, but they are not generally regarded as an actual disorder. However, BV is an indication that the defence against infection, which is partly conferred by a low pH, has been weakened and, under unfavourable circumstances, this leads to actual infection.

Shifts in the microbiota are associated with shifts in vaginal pH (Caillouette JC et al 1997). During BV the vaginal pH rises and a shift from being lactobacillus dominated to a biota in which *Gardnerella vaginalis* and anaerobic bacteria predominate is observed. The presence of anaerobic bacteria gives rise to offensive discharges and volatile amines at the increased pH (above 4.5).

The odour produced by an increased pH of vaginal fluid in BV is due to the presence of amines. The discharge in BV contains an increased concentration of several amines which are produced by anaerobic bacterial decarboxylation. Amines become volatile and thus odourous when they exist in the unprotonated form (free base). However, after addition of a strong acid the amines are converted to a protonated form (salt) and are not volatile. Lactic acid is a strong acid and thus suitable to diminish malodorous discharge.

By lowering the pH of the vagina with lactic acid it would make it easier for lactobacillus species to recolonize the vagina and thereby restore the natural resistance toward overgrowth by bacteria connected with BV. Lactate (i.e. salt of lactic acid) given in a gel containing growth substrate (glycogen) for lactobacillus has shown to be effective against elevated pH and bad odour in BV. (See EP 0 257 007)

The lactate-gel provides an acidic lactic acid cream containing glycogen without preservatives. Glycogen is found in abundance in the vaginal epithelial cells in fertile women and is an important nutrient substrate for the lactobacilli.

Compared with creams which contain only lactic acid or acidic creams with preservatives, the lactate-gel has been shown in vitro to have twice as favourable an effect on the survival of the lactobacilli. It can thus be used for re-establishing a normal environment in cases of BV.

The lactate gel has also been shown to have some antibacterial effect against certain BV-associated bacterial species due to its low pH. (See Andersch B et al; "Treatment of Bacterial Vaginosis with an Acid Cream: A Comparison between the Effect of Lactate-Gel and Metronidazole", Gynecol obstet Invest 21: 19-25 (1986)). However, no effects have been observed against yeast, such as *C*. *albicans* and *C*. *krusei.*

Conventional methods for treating fungal infections in the vagina generally involve the administration of antibiotics, e.g. imidazole derivatives. There are of course many incentives for reducing the use of antibiotics, e.g. due to the development of resistance.

Further, it is difficult to diagnose whether a patient suffers from BV or a fungal infection. This leads to the prescription of antibiotics as a matter of precaution, resulting in unnecessary excessive administration of antibiotics.

It would of course be very useful to obtain a non-antibiotic treatment, which beside having the abovementioned characterisics of lowering the vaginal pH and diminishing odour, also is effective for treating or preventing BV and/or fungal infections, particularly yeast infections. Ideally, the treatment should be effective irrespective of whether a patient suffers from, or is at risk of contracting, BV or fungal infection. Until now, no such possibility has been provided.

### Summary of the invention

An aim of the present invention is to provide a more efficient composition for the treatment of urogenital conditions, which lowers pH and is effective against bad odour, and which is also effective for treating bacterial vaginosis and fungal infections, in particular fungal infections caused by yeast species.

This aim is achieved by a composition for use according to claim 1.

The incorporation of LF, and/or a peptide fragment thereof, enhance the antimicrobial activity of the composition and it may thus be used for the purpose of inhibiting the growth of and/or killing undesired bacteria and fungi in the urogenital tract, in particular the vagina.

The concentration of LF and/or a peptide fragment thereof is in the range of 0.01 to 5 mg/ml, and said LF may be recombinant human LF. Said peptide fragment of lactoferrin may be synthetic or recombinant.

The concentration of lactic acid is at least 0.1% and < 1%.

The composition may further comprise a neutralizing substance, and the content of lactic acid and neutralizing substance is preferably such that the pH lies within the range of 3.5 to 4.5.

The composition may further comprise a growth substrate for lactic acid bacteria, and the weight ratio of lactic acid to growth substrate is preferably in the range of 20:1 to 500:1, more particularly about 50:1.

The composition may further comprise an inert vehicle and/or a consistency agent.

The composition may be e.g. in the form of a cream, a gel, a vaginal suppository, a tablet or a spray.

Furthermore, the invention relates to the use according to claim 17, and to the composition for use according to claim 22. The medicament has an antimicrobial activity.

The conditions to be treated and/or prevented comprises an elevated pH and/or a disturbed microbiota in the urogenital tract. A disturbed vaginal microbiota is characterized by abnormally high levels of BV-associated bacteria (anaerobic gram-negative bacteria, anaerobic gram-positive cocci, staphylococci streptococci, E. coli) and/or Candida, in combination with abnormally low levels of lactobacilli.

There are several advantages with the composition according to the present invention:
■ The composition is lenient to lactobacilli.
■ The combination of lactic acid and LF in a single composition gives rise to unexpected synergy effects, since it lowers the pH, diminishes odour and inhibits the growth of BV-associated bacteria and yeast.
■ The composition is effective irrespective of whether a patient suffers from BV or Candida-infection, which of course is an advantage if a reliable diagnosis cannot be established, in particular in the case of self-care.
■ The use of antibiotics may be reduced.

### Brief description of the figures

Fig 1 shows the antifungal activity of LF against *C*. *albicans* in the presence of lactate (a) and hydrogen chloride (b). LF was incubated together with various concentrations of lactate diluted in 0.2 % bactopeptone and *C.albicans* for 2 h. Thereafter each solution was analysed for viable counts.
Fig 2 shows the effects of LF (2 mg/ml) on two *Lactobacillus* strains commonly found in the vaginal microbiota. The bacteria were incubated for 3 h in various in various concentrations of lactate diluted in 0.34 % BHI and incubated in CO₂.
Fig 3 shows the antibacterial activity of LF and lactate on *L. gasseri* and *E. coli* (a comparative example) tested under the same conditions (0.34 % BHI, CO₂, 2 h of incubation).
Fig 4 shows the growth inhibition of *G*. *vaginalis* (Gv 14) and two *P*. *bivia* strains (Pb 57, Pb 62) in the presence of various concentrations of LF. The bacteria were incubated in 0,34% BHI under anaerobic conditions for 20 hours. The growth inhibition was analyzed spectrophotometrically.
Fig 5 shows the microbicidal activity of LF and rHLF on *C. albicans* (5a) and *E. coli* (a comparative example) (5b) in the presence of lactate (concentrations 5 and 1 %, diluted in 0.2 % bactopeptone). The solutions were incubated for 2 h and thereafter analysed by viable count.
Fig 6 shows the fungicidal activity of LF and rHLF on *C*. *albicans* in the presence of lactate (concentration 1 and 0.2 % in 0.2 % bactopeptone). The solutions were incubated for 2 and 20 h and analysed by viable counts.
Fig 7 shows the bactericidal activity of LF (2 mg/ml) and rHLF (2 mg/ml) in the presence of lactate (5, 1 and 0.2 % diluted in 0.2 % bactopeptone). The solutions were incubated for 2 h and analysed by viable counts.
Fig 8 shows the fungicidal activity of the LF peptide HL2 (250 ug/ml) in the presence of various concentrations of lactate (5, 1, 0.2, and 0.04 % diluted in 0.2 % bactopeptone). The solutions were incubated for 2 h and analysed by viable counts. The given percentage above the bars represents survival fraction of the inoculum.

### Detailed description of the invention

Lactoferrin (LF), which is described as an antibacterial agent, a modulator of the inflammatory response, and an immunoregulatory protein, is a single chain iron-binding glycoprotein. LF is found in colostrum and mature milk at levels of 2-7 g/l. In colostrum, LF makes up 43% of the total protein content. Other exocrine secretions like saliva, tears, bronchial mucus, and cervicovaginal fluid also contain LF (table 1).

It is remarkably resistant to proteolytic degradation by trypsin and trypsin-like enzymes. Pepsin digestion, however, give rise to a peptide fragment, lactoferricin, which is strongly bactericidal against a wide spectrum of bacteria *in vitro.* Both LF and lactoferricin are bactericidal and fungicidal *in vitro.*

**Table 1**

| **Levels of LF in some secretion** | | **mg/ml** |
|---|---|---|
| **Milk, human** | *colostrum* | 5-7 |
| | *mature milk* | 1-3 |
| | | |
| **Human secretions:** | | |
| | *tears* | 0.4-2.2 |
| | | |
| respiratory tract | *nasal secretion* | 0.1 |
| | *saliva* | 0.005-0.01 |
| | *bronchial mucus* | 0.03-0.04 |
| | | |
| genitourinary tract | *vaginal mucus* | 0.004-0.2 per mg protein |
| | *uterine secretions* | 0.5-1 |
| | *seminal plasma* | 0.4-2 |

It is of great importance to maintain the balance between the microorganisms in the vagina. In the treatment of BV (offensive discharges caused by imbalance in the vaginal biota without inflammation) it is important to re-establish the lactobacillus-dominated biota.

In the research work leading to the present invention, it was found that surprisingly good synergy effects could be obtained by combining the known lactate, described above, with LF and/or peptide fragments thereof, in accordance with claim 1.

Thus, the present invention provides an acidic lactic acid composition containing LF and/or peptide fragments thereof in accordance with claim 1. The composition according to the present invention reinforces the natural protection mechanisms of the vaginal mucosa by helping to re-establish the productive environment for lactobacilli, lowering the pH and also by exerting an antimicrobial activity on a number of microbes, e.g. *C*. *albicans*.

LF for use in the present invention may be human LF, recombinant human LF (rHLF), or bovine LF.

By human LF is meant LF purified from humans, e.g. from human milk.

By recombinant human LF is meant LF produced in pro- or eucaryotic cells (bacteria, fungal cells, plants, or animals) by hybride-DNA technology.

Peptide fragments of LF may also be used in the present invention, either alone or in combination with LF. It is possible to use either one kind of peptide fragment or two or more different kinds of peptide fragments.

By "peptide fragments out LF" is meant peptides of various sizes, based on the antimicrobial region of the LF molecule from the N-terminal end. The peptide fragment sequence is based on the sequence constituted of the amino acids (a.a.) 1-40, in particular the amino acids 12-40, of human LF counted from the N-terminal end. Synthetic as well as recombinant peptides may be used. An example of a LF peptide is lactoferricin. Another one could be the 25 or 23 amino acid long peptide starting from a.a.residue at position 16 or 18, respectively, from the N-terminal end. LF peptides suitable to be used in the present invention are described in the international application having publication number WO 00/01730.

The concentration of LF and/or a peptide fragment thereof is in the range of 0.01 to 5 mg/ml.

The concentration of lactic acid is at least 0.1% and < 1%.

Furthermore, lactate, i.e. salt of lactic acid, may be used in the present invention.

The composition may comprise a neutralizing substance for buffering the pH of the lactic acid. Examples of neutralizing substances are sodium hydroxide and ammonia.

The content of lactic acid and neutralizing substance may be such that the pH lies within the range from 3.5 to 4.5, preferably close to 3.8. The pH is advantageously not so low that the acid irritates the tissues or impairs the environment for the lactobacilli which require a moderately acid environment. A pH of 3.7-3.9 has been shown to be particularly advantageous.

The composition may further comprise a growth substrate for lactic acid bacteria. The growth substrate is preferably α(1-4) glucans containing α(1-6) branches.

An example of a suitable growth substrate is glycogen, which is found in abundance in the vaginal epithelial cells in fertile women and is an important nutrient substrate for lactobacilli. Glycogen is used as the growth substrate for the lactobacilli, so that the treatment not only results in adjustment of the pH level to a lower figure by means of supplying lactic acid, but also to re-establishment of an advantageous environment for the growth of the lactobacilli in order to regenerate the natural conditions. Other examples of growth substrates are lactose, dextrose, glucose, and amylopectin.

Lactic acid is incorporated in a considerably greater weight proportion than the growth substrate, for example in weight ratios of 20:1 to 500:1, in particular 50:1.

The composition according to the invention may be in the form of a cream, a gel, a spray, a tablet or a vaginal suppository. The composition according to the present invention consequently has a cream or gel consistency, a tablet consistensy, a consistency suitable for spray administration, or is given the form of a vaginal suppository or a tablet.

The composition according to the invention may be vaginally, perorally or transdermally administered.

A vehicle for the active constituents of the composition may be added to form a usable pharmaceutical product. The composition according to the present invention may contain vehicles which are of a type which is inert in this connection, and which provide cream- or gel-like consistency, or a consistency suitable for spray administration. Alternatively, the inert vehicle is a substance which melts at body temperature and in body fluid.

If a vaginal suppository is to be produced, a vehicle is required which makes it possible to form an article which is relatively solid at room temperature and in a dry environment, but which melts at body temperature and in contact with body fluid. In this connection there may be used polyethylene glycol, PEG, which gradually melts in the vaginal environment.

One suitable vehicle for a cream is propylene glycol, but other substances of an inert nature are also known to be of use in this connection as vehicles, e.g. triglycerides.

The composition according to the present invention may further comprise a consistency agent such as, in the case of creams and gels, methyl hydroxypropyl ether of cellulose. As an example of a commercial product that may be mentioned is Hypromellosum® 90 HG 4000. Another example of a consistency agents is corn starch.

By a "condition in the urogenital tract" is meant any disturbance or disorder caused by C. albicans in the urinary tract or in the genital organs in a female and/or male. In particular, the invention is suited for the treatment of conditions in the vagina.

A condition to be treated according to the invention may involve an elevated pH and/or a disturbed microbiota in the urogenital tract. By an "elevated pH" is meant a pH above 4.5.

By a "disturbed microbiota" is meant a biota having abnormally high levels of BV-associated bacteria, such as *G. vaginalis,* anaerobic gram-negative bacteria (*Prevotella-, Bacteroides-,* and *Fusobacterium* species), anaerobic gram-positive cocci, *Mobiluncus* species, and/or abnormally high levels of *E. coli,* staphylococci (*S*. *aureus*), and streptococci, all in combination with abnormally low levels of lactobacilli. Also high levels of *Candida,* which may result in vaginitis are considered a disturbed vaginal microbiota.

By an "abnormally high level" is meant a level which is 100 to 1000 fold higher than the level in a normal person.

By an "abnormally low level" is meant not predominating, i.e. not present or less than one lactobacillus morphotype per microscopical immersion field, or less or equal to other morphotypes per immersion field. (Assessment according to the "Nugent scoring system", used internationally to describe bacterial imbalance in vaginal secretion. See Nugent RP et al 1991, J Clin Microbiol 29:297-301*.*)

By an "antimicrobial activity" is meant a bacteriostatic, fungistatic, bactericidal and/or fungicidal activity, i.e. the ability to retard the growth of and kill certain bacteria and fungi, respectively.

By "prophylaxis and/or treatment of a condition" is meant any treatment in order to cure or alleviate a condition according to the above, or to prevent the development of such a condition.

Examples of conditions to be treated with the composition according to the invention are bacterial vaginosis, intermediate vaginal microbiota, and yeast infections, e.g. Candida-infections (Candida vaginitis, Candida balanitis).

By a "pharmaceutically effective amount" is meant an amount of the composition according to the invention, which will lead to the desired pharmacological and/or therapeutic effect. The desired pharmacological and/or therapeutic effect is, as stated above, to cure or alleviate and/or prevent the development of conditions in the urogenital tract, caused by C. albicans.

By a "patient" is meant any human or non-human mammal, female or male, in need of being treated with the composition and/or method according to the invention.

The composition according to the invention may further contain other substances, such as adjuvants, carriers, preservatives, vitamins, minerals, oestrogen etc, which are well known to persons skilled in the art.

Furthermore, it is possible to combine the composition according to the invention with other conventional pharmacological treatments of conditions in the urogenital tract, e.g. the treatment with antibiotics and/or antimycotics, such as imidazol preparations.

The composition may be in the form of a concentrate, intended to be diluted by the user before use. Such a concentrate is suitably diluted with e.g. pure water, aqueous solutions or saline.

Specific examples of a composition emerges from the following examplary compositions.

**Cream**

| | |
|---|---|
| Lactic acid | 5.0 g |
| NaOH ad. pH 3.9 | |
| Glycogen | 0.1 g |
| Propylene glycol (85%, remainder H₂O) | 15.0 g |
| Methylhydroxypropyl ether of cellulose e.g. Hypromellosum® 90 | ad. 100 g |
| HG 4000 (2.5%, remainder H₂O) | |
| rhLF | 0,2 g |

**Cream**

| | |
|---|---|
| Lactic acid | 5.0 g |
| Na OH (5M) | 4.1 g |
| Glycogen | 0.1 g |
| Propylene glycol (85%, remainder H₂O) | 15.0 g |
| Methylhydroxypropyl ether of cellulose e.g. Hypromellosum® 90 | ad. 100.0 g |
| HG 4000 (2.5%, remainder H₂O | |
| rhLF | 0,2 g |

### Examples

### Material and methods

### Bacterial and fungal strains

The bacterial strains *S. aureus* 1800 (CCUG 1800), *E. coli* 06, and the yeast *C. albicans* (ATCC 64549) have been used in the microplate assay. All microorganisms were cultured in brain heart infusion medium (BHI) on a shaker over night at 37°C. A volume of the culture was transferred to a new tube with BHI and incubated for two more h on the shaker. The bacteria were harvested in the log phase, washed once and thereafter suspended in the broth used for the microplate assay. The suspension was adjusted spectrophotometrically and diluted to a concentration of approximately 4 x 10⁶ cells per ml at 650 nm.

*L. gasseri* and *L*. *jensenii* were cultured on chocolate-agar plates for 24 h in 5 % CO₂/95 % air. They were thereafter harvested and added to BHI (0.34 %). After washing the bacterial solutions were adjusted spectrophotometrically and diluted to 8x10⁶ bacteria /ml.

*G. vaginalis* and *P. bivia* were cultured on chocolate-GL-plates for 48 h in anaerobic milieu. The bacteria were harvested and suspended in BHI for further incubation (4 hours at 37°C). The bacteria were washed twice in Bactopeptone (BP, 0,2 %). The concentration was adjusted spectrophotometrically and diluted to 1 x 10⁷ bacteria per ml.

### LF and peptide

LF from human milk was purchased from Sigma (Saint Louis, USA). Recombinant human LF was obtained from Agennix. A peptide fragment composed of 23 amino acids based on the antimicrobial region of human LF was synthesized by the fmoc-strategy.

### Minimum microbicidal and inhibitory concentrations, MMC.

Washed cells were suspended in 0.2 % BP (pH 7.0) or BHI medium diluted 1/10 (0.34%, pH 6.7-6.9) (Difco, USA). The concentration of bacterial or fungal cells was spectrophotometrically adjusted. LF or peptide, serially diluted in BHI_{dil} or BP by twofold steps (unless otherwise stated), were added in triplicate to the wells of a microtiterplate (200 ul per well). The bacterial or yeast cell solutions were added in 10 ul volumes to give a final concentration of approximately 2 x10⁵ cells per ml. The concentration of the stock solution was always checked by viable counts. The microplate was incubated at 37°C in a humid chamber for 2 h unless otherwise stated. Five ul were taken from each well and added as a drop onto a blood agar plate and incubated over night at 37°C. In some experiments more thorough viable counts were performed and the killing was expressed as remaining colony forming units in relation to the inoculum. The lower limit for detection of microorganisms was 200 CFU/ml.

The procedure for the microbicidal assay with regard to *G. vaginalis* and *P. bivia* was as described above except that the microplate was incubated under anaerobic conditions (2 h). Thereafter droplets of the solutions were analyzed for viable bacteria on blood agar plates after 48 h of anaerobic incubation at 37°C.

### Example 1

### Antimicrobial activity of lactate and LF

The antimicrobial activity of human LF was analysed by the microbicidal assay described above. Different microbes such as *C. albicans, E. coli* and *S. aureus* were analysed with respect to their survival in lactate (not containing the inert gel base, which makes the gel unsuitable for the microbicidal assay) containing LF.

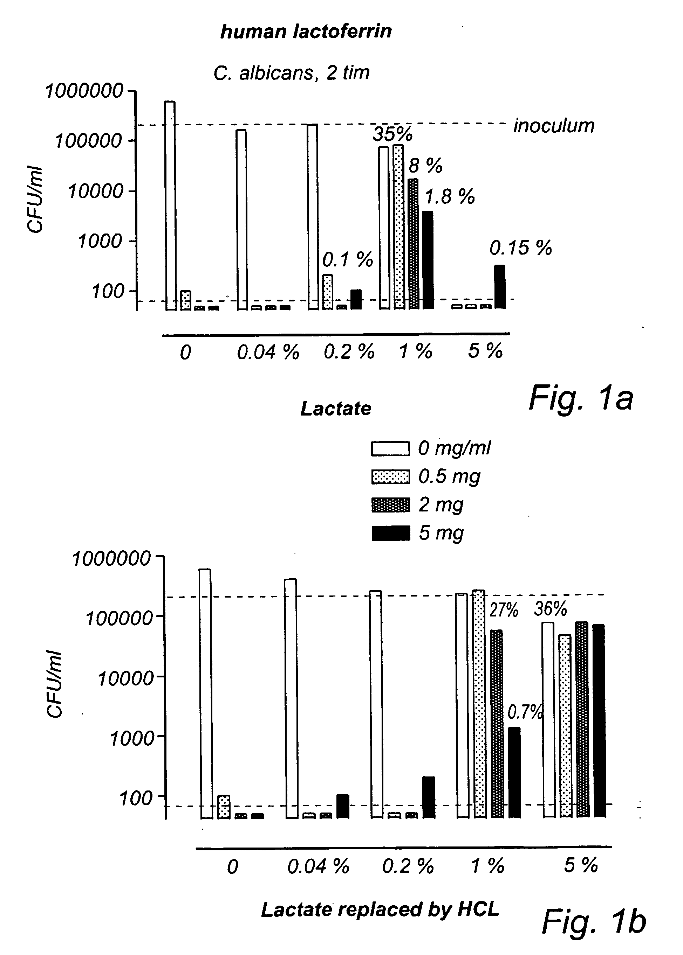

Fig 1 shows the antifungal activity of LF against *C. albicans* in the presence of lactate (a) and hydrogen chloride (b). LF was incubated together with various concentrations of lactate diluted in 0.2 % bactopeptone and *C. albicans* for 2 h. Thereafter each solution was analysed for viable counts.

Our results showed that LF enhanced the antimicrobial effect of lactate against *C. albicans* when lactate was diluted fivefold or more (< 1 %) for all LF concentrations except 0.5 mg at dilution 1 % of lactate (Fig 1a). LF reduced the yeast cells with 99.9% in the lactate concentrations 0.2 %, 0.04 % and 0 % lactate. In undiluted lactate an antifungal activity was observed.Lactate of 1% reduced the *Candida* by 65 %. However, in the presence of LF 92 and 98.2 % of the inoculate were killed with 2 and 5 mg /ml, respectively. Replacing the lactate by HCl giving the same pH as lactate resulted in reduced Candidacidal activity when undiluted (fig 1b). These experiments also showed that not only a low pH but also the lactate by itself was important for the antifungal activity (at undiluted lactate solution) .

The antibacterial activity of lactate towards *E. coli* and *S. aureus* was good both in the absence or presence of LF (Table 2 and 3).

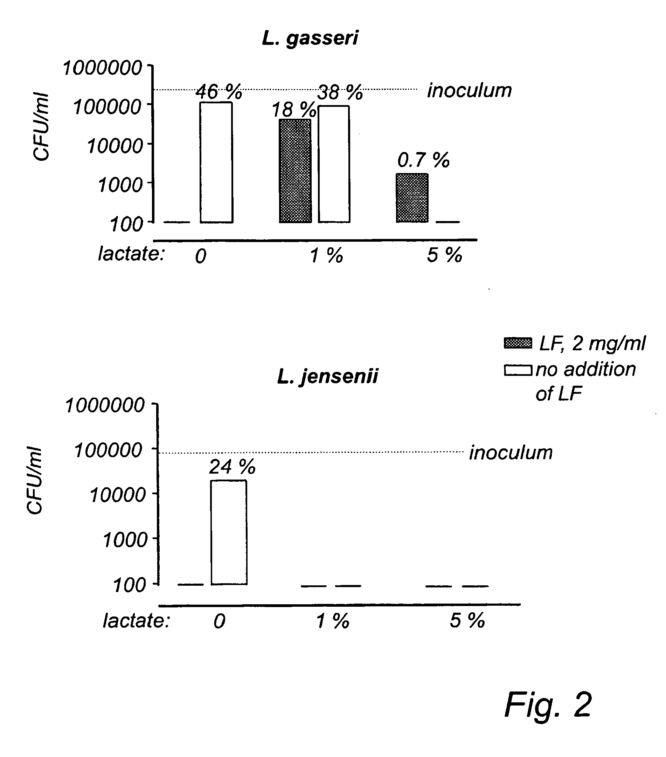

The effects of LF and lactate was also analysed with two Lactobacillus strains (*L*. *gasseri* and *L*. *jensenii*) frequently found in the vagina (fig 2).

Fig 2 shows the effects of LF (2 mg/ml) on *L. gas*seri and *L. jensenii* The bacteria were incubated for 3 h in various concentrations of lactate diluted in 0.34 % BHI and incubated in CO₂. *L. gasseri* survived better in the assay system than did *L. jensenii.* Undiluted lactate (5%) affected the survival of *Lactobacillus.* In 1 % of lactate *L. jensenii* did not survive, while 18% of the LF treated *L. gasseri* survived.

In a comparative experiment a comparison between *E. coli* and *L. gasseri* was done in order to find out if different incubation conditions (0.34 % BHI, CO₂ incubator) would change the effects of LF. Thus *E. coli* was treated in the same way as *L*. *gasseri.*

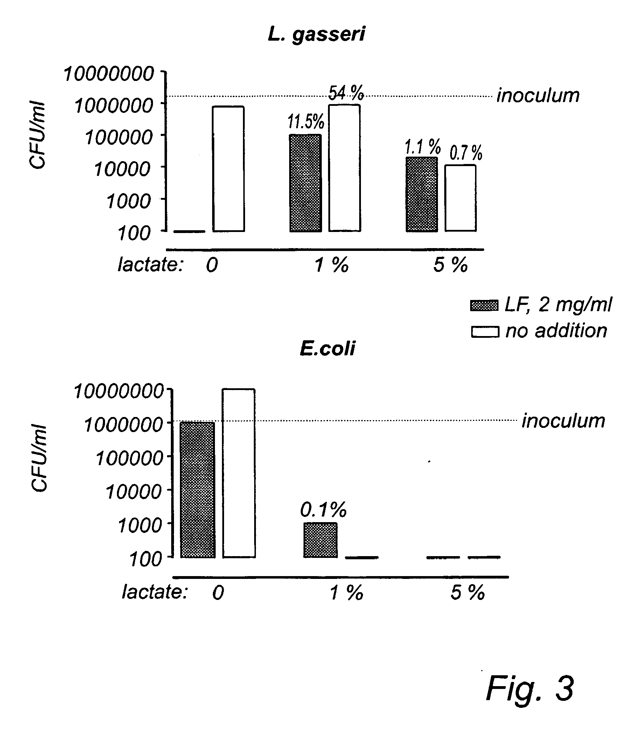

As found earlier *E. coli* was sensitive for lactate, and *L. gasseri* was more resistant than *E. coli* in lactate with or without LF (fig 3).

**Table 2 (a comparative example)**

| | | | | | |
|---|---|---|---|---|---|
| Killing activity of LF in various concentrations of lactate as analysed by the microbicidal microplate- droplet assay. Figures represent percentage of bacteria remaining in relation to the inoculum. +++ means that the number of colony forming units (CFU) contained within 5 ul of a droplet formed a smooth layer of bacteria, ++ means that a "ruffled" area is seen, and finally 0 means that no CFU were found in triplicate samples of 5 ul (> 99.95 killed of the inoculum). | | | | | |

| *E. coli* killing- | | | | | |
|---|---|---|---|---|---|
| | | HLF, ug/ml | | | |
| Time of incubation | lactate concentration | 500 | 2000 | 5000 | 0 |
| ***2 h*** | 5% | 0 | 0 | 0 | 0 |
| | 1 % | ++ | (+++) | (+++) | (+++) |
| | 0.2 % | ++ | (+++) | ++ | ++ |
| | no lactal | +++ | ++ | ++ | +++ |
| | | | | | |
| ***5 h*** | 5 % | 0 | 0 | 0 | 0 |
| | 1% | 5 | 9 | 16 | 6 |
| | 0.2 % | 4 | 5 | 0.4 | 5 |
| | no lactal | +++ | 3 | 2 | +++ |

**Table 3 (a comparative example)**

| | | | |
|---|---|---|---|
| Killing activity of LF in various concentrations of lactate as analysed by the microbicidal microplate- droplet assay. Figures represent percentage of bacteria remaining in relation to the inoculum. +++ = the number of colony forming units contained within 5 ul of a droplet formed a smooth layer of bacteria. (parenthesis = a tendency towards a ruffled area compared with the control). 0 = no bacteria in triplicate samples of 5 ul (> 99.95 killed of the inoculum). | | | |

| ***S. aureus* killing** | | | |
|---|---|---|---|
| Time of incubation | lactate | HLF, ug/ml | |
| | | 500 | t0 |
| ***2 h*** | 5 % | 0 | 0 |
| | 1 % | 0 | 0 |
| | 0.2 % | 0 | 0 |
| | no lactal | (+++) | +++ |
| | | | |
| ***5 h*** | 5 % | 0 | 0 |
| | 1 % | 0 | 0 |
| | 0.2 % | 0 | 0 |
| | no lactal | 0.3 | +++ |

The experiment indicated that *L. gasseri* was more resistant than *E. coli* to the effects of lactate and LF in combination, despite the poor survival of *L.gasseri* in LF without lactate.

The antibacterial activity of LF on *G. vaginalis* and *P. bivia* was also analysed. In this experiment the bacterial strains did not survive in our test system, despite the anaerobic condition. (See example 2 below for further experiments)

In summary, LF enhanced the antimicrobial activity of lactate against *C. albicans,* and *E. coli.* (although the magnitude of the activity was somewhat dependent on the LF as well as lactate concentrations regarding *C. albicans*).

At the undiluted lactate concentration the antimicrobial activity was complete without LF when analysed against *C. albicans, S. aureus,* and *E. coli.* It can also be concluded that it is not only the pH, which is important for the antimicrobial activity, but also the type of molecule giving rise to the low pH. Thus the α-hydroxipropionic acid, lactate, is more active in the presence of LF than HCl (fig 1).

It may seem that LF in combination with lactate could be beneficial for the host against both unpleasant odours and antimicrobial imbalance.

### Example 2 (a comparative example)

### Antimicrobial activity of LF on G. vaginalis and P. bivia.

The microbicidal assay was modified in order to obtain survival conditions for *G. vaginalis* and *P. bivia.* Instead of 0.2 % BP 0.34% BHI was used. The bacterial strains were analyzed with only LF added in various concentrations to the wells (no lactate present). The plate was incubated for 20 hours.

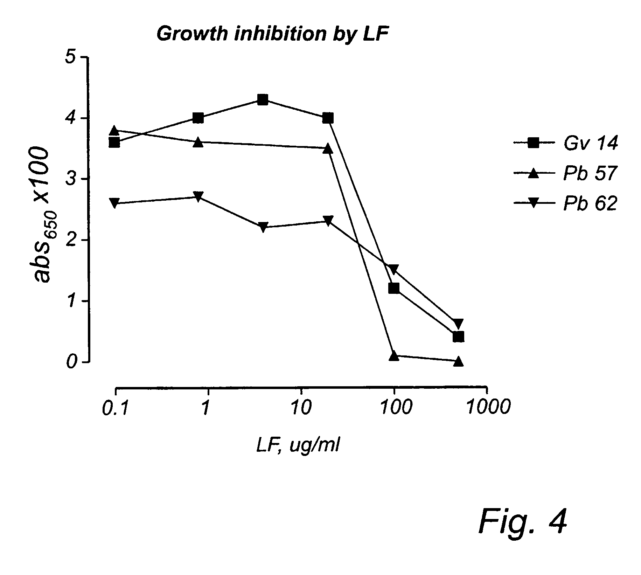

Growth inhibition by LF was seen for all strains tested (fig 4).

In a second series of experiment the minimal bactericidal concentration (MBC) at 99 % killing was measured for seven strains of *G. vaginalis* (table 4). Out of those only one showed some growth when incubated with 2.5 mg of LF. Thus, an antimicrobial activity of LF on *P.bivia* and *G. vaginalis* as well was confirmed.

**Table 4**

| | | |
|---|---|---|
| Bactericidal activity of LF. LF was diluted with fivefold serial steps. The concentration at which a 99% killing was obtained was defined as the MBC₉₉. | | |

| **Bacterial strain** | | **MBC₉₉** **ug/ml** |
|---|---|---|
| G. vaginalis, strain code: | | |
| | 17 | >2500 |
| | 90 | 4 |
| | 23 | 1000 |
| | 12 | 500 |
| | 96 | 100-500 |
| | 54 | 20 |
| | 51 | 100 |

| | | |
|---|---|---|
| The concentrations used were 2500, 500, 100, 20, 4 ug/ml | | |

### Example 3 (a comparative example)

### Antimicrobial activity of lactate and rhLF

The aim was to test whether rHLF would enhance the antimicrobial effects of lactate to the same extent as LF purified from human milk (Sigma).

The antimicrobial activity of lactate (not including gel base) containing rHLF was analysed by the microplate assay. The microorganisms *C. albicans, E. coli* and *S. aureus* were analysed.

In our first experiment rHLF and LF were compared under conditions without lactate (not shown). Analysis with the microplate assay showed that both LF preparations were similar in their microbicidal activity against *E. coli,* while LF was somewhat more active against S. aureus and *C. albicans* at concentrations of 0.25 and 0.125 mg/ml (2 h of incubation).

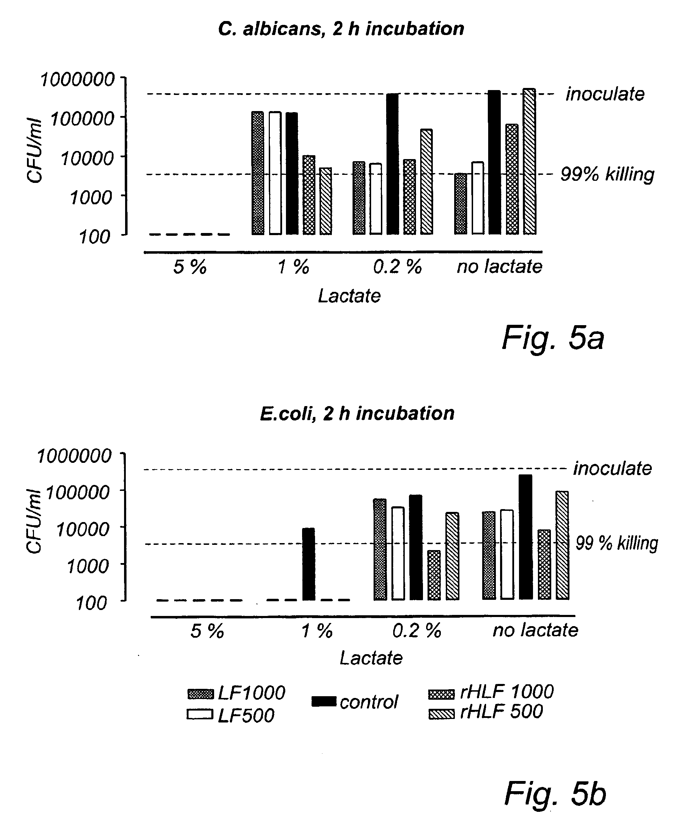

In the presence of lactate the strongest activities against *C. albicans* were obtained with undiluted lactate (5 %), since no yeast cells survived (fig 5 a). However, at a lactate concentration of 1 %, rHLF (0.5-1.0 mg/ml) almost reached 99% killing of the inoculate. At a concentration of 0.2 % lactate 1 mg/ml of either of the LF preparations almost reached 99% killing. With regard to killing of *E. coli,* no bacteria survived at undiluted lactate (5 %), while both LF preparations enhanced the killing at a lactate concentration of 1 % (fig 5 b). rHLF at 1 mg/ml was the most effective of the two LFs at the lactate concentration 0.2 % and in lactate free solution.

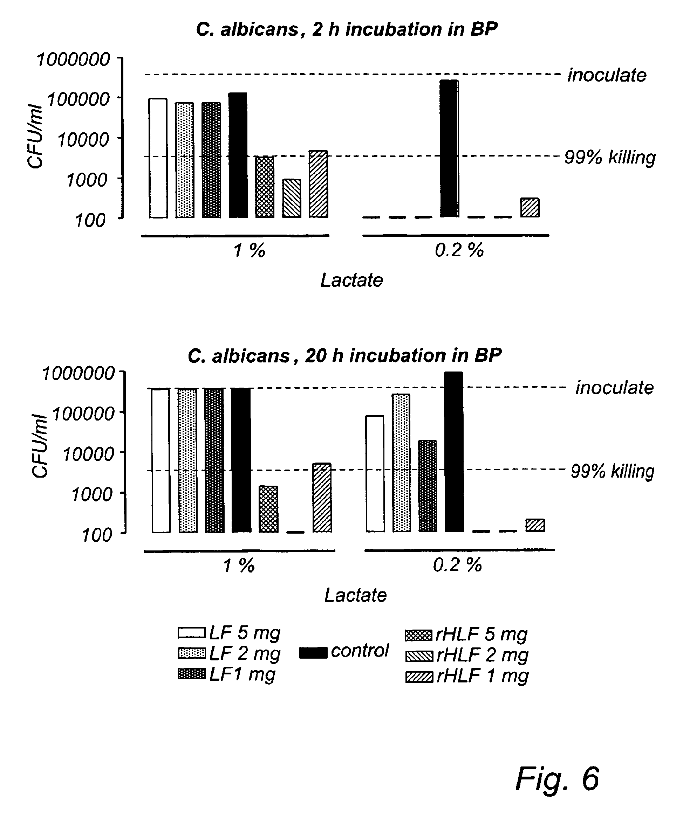

In the following experiment higher concentrations of LF and rhLF were studied with regard to fungicidal effect on *C. albicans* (fig 6). Once again, a stronger effect was obtained with rHLF in comparison to LF at the lactate concentrations 1 %. At 0.2 % of lactate, the stronger effect of rHLF was observed only after a longer incubation time (20 h, fig 6).

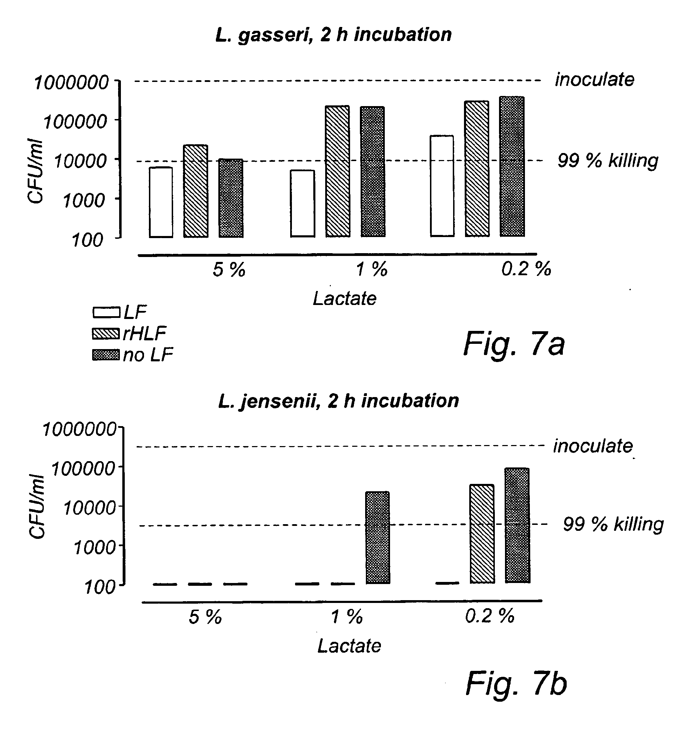

Based on the results obtained with 2 mg of rHLF (see fig 6), the LF preparations were analysed for their effects on the two Lactobacillus species from the vaginal microbiota, *L. gasseri* and *L. jensenii* (fig 7). At 2 h of incubation, rHLF had no additional effects on *L. gasseri* survival than that exerted by the presence of lactate (fig 7a), while both LF preparations enhanced the killing of *L. jensenii* at 1 % lactate. At 0.2% lactate *L. jensenii* was more resistant to the antibacterial effect of rhLF. Thus, *L. jensenii* was more susceptible to the effects of the LF preparations than *L. gasseri,* particularly at 1 % of lactate (fig 7b), and both lactobacillus species were more resistant to rhLF.

To summarize, synergy effects were obtained by adding rHLF to the lactate. rHLF appeared somewhat weaker in its antimicrobial activity compared with LF when tested in a weak BHI medium (0.34 %). In contrast, rHLF was somewhat stronger than LF in the presence of lactate (1 or 0.2 %), except when using lactobacilli. This opposite effect on lactobacilli is beneficial for the host, since lactobacilli belong to the healthy normal microbiota. The results indicate that 2 mg/ml of rHLF could be a suitable concentration in lactate.

### Example 4

### Antimicrobial activity of lactate and a LF peptide, HL2.

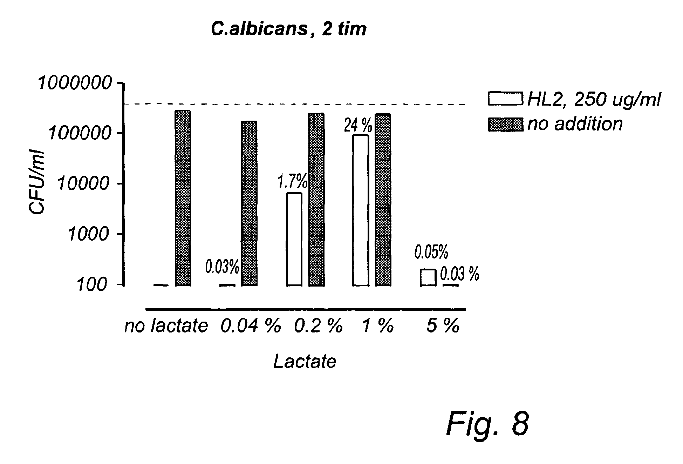

A peptide fragment based on the antimicrobial region of the LF molecule containing 23 a.a. was anlysed for its fungicidal activity in the presence of lactate. As seen in fig 8, the addition of HL2 reduced the yeast cell viability at all lactate concentrations as well as when no lactate was added. At the undiluted lactate concentration (5 %), however, the effect of the lactate was complete even in the absence of HL2.
Thus, a short LF peptide fragment added antifungal activity to the lactate solutions.

### Summary

To summarize the examples, the addition of LF to lactate enhanced the antimicrobial effect of it.

Regarding the antifungal activity of lactate on *Candida,* the addition of LH and particularly rHLF to the lactate showed synergistic effects (fig 5a and fig 6).

This synergy effect was unexpected since the addition of negatively charged molecules (lactate) would be expected to compete with the negatively charged groups on the bacterial surface for the interaction with the cationic LF. It is generally believed that electrostatic interaction with the negatively charged surfaces of microorganisms is the first step in the microbicidal activity of LF. For instance, it has been shown that increasing concentrations of phosphate and bicarbonate may decrease the fungicidal activity of human LF (Soukka et al., 1992, FEMS Microbiology letters 90:223-228).

It should also be emphasized that lactobacilli such as *L. gasseri* unexpectedly survived equally well in the lactate whether or not rHLF (fig 7a) had been added.

## Claims

1. A composition comprising lactic acid, and/or a salt thereof, and lactoferrin, and/or a peptide fragment thereof, which peptide fragment is based on the antimicrobial region of the lactoferrin molecule from the N-terminal end, for use in the treatment and/or prophylaxis of conditions in the urogenital tract caused by C. albicans, **characterized in that** the concentration of lactoferrin, and/or a peptide fragment thereof, is in the range of 0.01 to 5 mg/ml, and the concentration of lactic acid, and/or a salt thereof, is at least 0.1 % and < 1 %.

2. A composition for use according to claim 1, wherein said lactoferrin is recombinant human lactoferrin.

3. A composition for use according to any one of the preceding claims, wherein said peptide fragment of lactoferrin is synthetic or recombinant.

4. A composition for use according to any one of the preceding claims, further comprising a neutralizing substance.

5. A composition for use according to claim 4, wherein the content of lactic acid, and/or a salt thereof, and neutralizing substance is such that the pH lies within the range of 3.5 to 4.5.

6. A composition for use according to claim 4 or 5, wherein said neutralizing substance is sodium hydroxide.

7. A composition for use according to any one of the preceding claims, further comprising a growth substrate for lactic acid bacillus species.

8. A composition for use according to claim 7, wherein the weight ratio of lactic acid, and/or a salt thereof, to growth substrate is in the range of 20:1 to 500:1.

9. A composition for use according to claim 8, wherein the weight ratio of lactic acid, and/or a salt thereof, to growth substrate is 50:1.

10. A composition for use according to any one of the claims 7-9, wherein said growth substrate is α(1-4) glucans containing α(1-6) branches.

11. A composition for use according to claim 10, wherein said growth substrate is selected from the group consisting of glycogen, amylopectin, glucose, dextrose, and lactose.

12. A composition for use according to any one of the preceding claims, further comprising an inert vehicle.

13. A composition for use according to claim 12, wherein said inert vehicle is propylene glycol or polyethylene glycol.

14. A composition for use according to any one of the preceding claims, further comprising a consistency agent.

15. A composition for use according to claim 14, wherein said consistency agent is methyl hydroxypropyl ether of cellulose.

16. A composition for use according to any one of the preceding claims, in the form of a cream, a gel, a vaginal suppository, a tablet or a spray.

17. Use of a composition according to any one of the claims 1-16, for the preparation of a medicament for the prophylaxis and/or treatment of a condition in the urogenital tract caused by C. albicans.

18. Use according to claim 17, wherein said medicament has an antimicrobial activity.

19. Use according to claim 17 or 18, wherein said condition involves an elevated pH and/or a disturbed microbiota in the urogenital tract.

20. Use according to claim 19, wherein said disturbed microbiota is **characterized by** abnormally high levels of BV-associated bacteria (anaerobic gram-negative bacteria, anaerobic gram-positive cocci, staphylococci streptococci, E. coli) and/or Candida, in combination with abnormally low levels of lactobacilli.

21. Use according to any one of the claims 17-20, wherein said condition is selected from the group consisting of bacterial vaginosis, intermediate vaginal microbiota, and yeast infections.

22. A composition for use according to any one of the claims 1-16 for use in the prophylaxis and/or treatment of a condition in the urogenital tract caused by C. albicans.

23. A composition for use according to claim 22, wherein said composition has an antimicrobial activity.

24. A composition for use according to claim 22 or 23, wherein said condition involves an elevated pH and/or a disturbed microbiota in the urogenital tract.

25. A composition for use according to claim 24, wherein said disturbed microbiota is **characterized by** abnormally high levels of BV-associated bacteria (anaerobic gram-negative bacteria, anaerobic gram-positive cocci, staphylococci streptococci, E. coli) and/or Candida, in combination with abnormally low levels of lactobacilli.

26. A composition for use according to any one of the claims 22-25, wherein said condition is selected from the group consisting of bacterial vaginosis, intermediate vaginal microbiota, and yeast infections.

## Patentansprüche

1. Zusammensetzung, die Milchsäure und/oder ein Salz davon und Laktoferrin und/oder ein Peptidfragment davon aufweist, wobei das Peptidfragment auf der antimikrobiellen Region des Laktoferrinmoleküls aus dem N-terminalen Ende basiert, zur Verwendung bei der Behandlung und/oder Prophylaxe von Erkrankungen im Urogenitaltrakt, die von C. albicans verursacht werden, **dadurch gekennzeichnet, dass** die Konzentration von Laktoferrin und/oder einem Peptidfragment davon im Bereich von 0,01 bis 5 mg/ml liegt und die Konzentration von Milchsäure und/oder einem Salz davon mindestens 0,1 % und < 1 % beträgt.

2. Zusammensetzung zur Verwendung nach Anspruch 1, wobei es sich bei dem Laktoferrin um rekombinantes humanes Laktoferrin handelt.

3. Zusammensetzung zur Verwendung nach einem der vorherigen Ansprüche, wobei das Peptidfragment von Laktoferrin synthetisch oder rekombinant ist.

4. Zusammensetzung zur Verwendung nach einem der vorherigen Ansprüche, die des Weiteren eine neutralisierende Substanz aufweist.

5. Zusammensetzung zur Verwendung nach Anspruch 4, wobei der Gehalt von Milchsäure und/oder einem Salz davon und der neutralisierenden Substanz derart ist, dass der pH-Wert im Bereich von 3,5 bis 4,5 liegt.

6. Zusammensetzung zur Verwendung nach Anspruch 4 oder 5, wobei es sich bei der neutralisierenden Substanz um Natriumhydroxid handelt.

7. Zusammensetzung zur Verwendung nach einem der vorherigen Ansprüche, die des Weiteren ein Wachstumssubstrat für Milchsäure-Bazillus-Arten aufweist.

8. Zusammensetzung zur Verwendung nach Anspruch 7, wobei das Gewichtsverhältnis von Milchsäure und/oder einem Salz davon zu Wachstumssubstrat im Bereich von 20:1 bis 500:1 liegt.

9. Zusammensetzung zur Verwendung nach Anspruch 8, wobei das Gewichtsverhältnis von Milchsäure und/oder einem Salz davon zu Wachstumssubstrat 50:1 beträgt.

10. Zusammensetzung zur Verwendung nach einem der Ansprüche 7-9, wobei es sich bei dem Wachstumssubstrat um α(1-4)Glukan handelt, das α(1-6)-Verzweigungen aufweist.

11. Zusammensetzung zur Verwendung nach Anspruch 10, wobei das Wachstumssubstrat aus der Gruppe ausgewählt ist, die aus Glykogen, Amylopektin, Glukose, Dextrose und Laktose besteht.

12. Zusammensetzung zur Verwendung nach einem der vorherigen Ansprüche, die des Weiteren ein inertes Vehikel aufweist.

13. Zusammensetzung zur Verwendung nach Anspruch 12, wobei es sich bei dem inerten Vehikel um Propylenglykol oder Polyethylenglykol handelt.

14. Zusammensetzung zur Verwendung nach einem der vorherigen Ansprüche, die des Weiteren ein Konsistenzmittel aufweist.

15. Zusammensetzung zur Verwendung nach Anspruch 14, wobei es sich bei dem Konsistenzmittel um Methylhydroxypropylether von Zellulose handelt.

16. Zusammensetzung zur Verwendung nach einem der vorherigen Ansprüche in der Form einer Creme, eines Gels, eines Vaginalzäpfchens, einer Tablette oder eines Sprays.

17. Verwendung einer Zusammensetzung nach einem der Ansprüche 1-16 für die Zubereitung eines Medikaments für die Prophylaxe und/oder Behandlung einer Erkrankung im Urogenitaltrakt, die von C. albicans verursacht wird.

18. Verwendung nach Anspruch 17, wobei das Medikament eine antimikrobielle Aktivität aufweist.

19. Verwendung nach Anspruch 17 oder 18, wobei die Erkrankung mit einem erhöhten pH-Wert und/oder einer gestörten Biozönose im Urogenitaltrakt einhergeht.

20. Verwendung nach Anspruch 19, wobei die gestörte Biozönose von anomal hohen Konzentrationen von BV-assoziierten Bakterien (anaerobe gramnegative Bakterien, anaerobe grampositive Kokken, Staphylokokken, Streptokokken, E. coli) und/oder Candida in Kombination mit anomal niedrigen Konzentrationen von Laktobazillen **gekennzeichnet** ist.

21. Verwendung nach einem der Ansprüche 17-20, wobei die Erkrankung aus der Gruppe ausgewählt ist, die aus bakterieller Vaginose, intermediärer vaginaler Biozönose und Hefeinfektionen besteht.

22. Zusammensetzung zur Verwendung nach einem der Ansprüche 1-16 für die Verwendung bei der Prophylaxe und/oder Behandlung einer Erkrankung im Urogenitaltrakt, die von C. albicans verursacht wird.

23. Zusammensetzung zur Verwendung nach Anspruch 22, wobei die Zusammensetzung eine antimikrobielle Aktivität aufweist.

24. Zusammensetzung zur Verwendung nach Anspruch 22 oder 23, wobei die Erkrankung mit einem erhöhten pH-Wert und/oder einer gestörten Biozönose im Urogenitaltrakt einhergeht.

25. Zusammensetzung zur Verwendung nach Anspruch 24, wobei die gestörte Biozönose von anomal hohen Konzentrationen von BV-assoziierten Bakterien (anaerobe gramnegative Bakterien, anaerobe grampositive Kokken, Staphylokokken, Streptokokken, E. coli) und/oder Candida in Kombination mit anomal niedrigen Konzentrationen von Laktobazillen **gekennzeichnet** ist.

26. Zusammensetzung zur Verwendung nach einem der Ansprüche 22-25, wobei die Erkrankung aus der Gruppe ausgewählt ist, die aus bakterieller Vaginose, intermediärer vaginaler Biozönose und Hefeinfektionen besteht.

## Revendications

1. Composition comprenant de l'acide lactique, et/ou un sel de celui-ci, et de la lactoferrine, et/ou un fragment peptidique de celle-ci, lequel fragment peptidique est basé sur la région antimicrobienne de la molécule de la lactoferrine de l'extrémité N-terminale, destinée à être utilisée dans le traitement et/ou la prophylaxie de conditions affectant le tractus urogénital et causées par C. albicans, **caractérisée en ce que** la concentration en lactoferrine, et/ou en un fragment peptidique de celle-ci, se trouve dans la plage de 0,01 à 5 mg/mL, et la concentration en acide lactique, et/ou en un sel de celui-ci, est d'au moins 0,1 % et < 1%.

2. Composition destinée à être utilisée selon la revendication 1, dans laquelle ladite lactoferrine est de la lactoferrine humaine recombinante.

3. Composition destinée à être utilisée selon l'une quelconque des revendications précédentes, dans laquelle ledit fragment peptidique de la lactoferrine est synthétique ou recombinant.

4. Composition destinée à être utilisée selon l'une quelconque des revendications précédentes, comprenant en outre une substance neutralisante.

5. Composition destinée à être utilisée selon la revendication 4, dans laquelle la teneur en acide lactique, et/ou en un sel de celui-ci, et en substance neutralisante, est telle que le pH se trouve dans la plage de 3,5 à 4,5.

6. Composition destinée à être utilisée selon la revendication 4 ou 5, dans laquelle ladite substance neutralisante est l'hydroxyde de sodium.

7. Composition destinée à être utilisée selon l'une quelconque des revendications précédentes, comprenant en outre un substrat de croissance pour les espèces bacillus d'acide lactique.

8. Composition destinée à être utilisée selon la revendication 7, dans laquelle le rapport en poids de l'acide lactique, et/ou d'un sel de celui-ci, sur le substrat de croissance, se trouve dans la plage de 20/1 à 500/1.

9. Composition destinée à être utilisée selon la revendication 8, dans laquelle le rapport en poids de l'acide lactique, et/ou d'un sel de celui-ci, sur le substrat de croissance est de 50/1.

10. Composition destinée à être utilisée selon l'une quelconque des revendications 7 à 9, dans laquelle ledit substrat de croissance est les α(1-4)glucanes contenant des ramifications α(1-6).

11. Composition destinée à être utilisée selon la revendication 10, dans laquelle ledit substrat de croissance est choisi dans le groupe constitué par le glycogène, l'amylopectine, le glucose, le dextrose, et le lactose.

12. Composition destinée à être utilisée selon l'une quelconque des revendications précédentes, comprenant en outre un véhicule inerte.

13. Composition destinée à être utilisée selon la revendication 12, dans laquelle ledit véhicule inerte est le propylène glycol ou le polyéthylène glycol.

14. Composition destinée à être utilisée selon l'une quelconque des revendications précédentes, comprenant en outre un agent de consistance.

15. Composition destinée à être utilisée selon la revendication 14, dans laquelle ledit agent de consistance est le méthyl hydroxypropyl éther de cellulose.

16. Composition destinée à être utilisée selon l'une quelconque des revendications précédentes, sous la forme d'une crème, d'un, gel, d'un suppositoire vaginal, d'un comprimé ou d'un spray.

17. Utilisation d'une composition selon l'une quelconque des revendications 1 à 16, pour la préparation d'un médicament destiné à la prophylaxie et/ou au traitement d'une condition affectant le tractus urogénital et causée par C. albicans.

18. Utilisation selon la revendication 17, dans laquelle ledit médicament a une activité antimicrobienne.

19. Utilisation selon la revendication 17 ou 18, dans laquelle ladite condition implique un pH élevé et/ou un microbiote perturbé dans le tractus urogénital.

20. Utilisation selon la revendication 19, dans laquelle ledit microbiote perturbé est **caractérisé par** des niveaux anormalement élevés de bactéries associées aux BV (vaginoses bactériennes) (bactéries anaérobies gram négatif, cocci anaérobies gram positif, staphylocoques, streptocoques, E. coli) et/ou de Candida, en combinaison avec des niveaux anormalement bas de lactobacilles.

21. Utilisation selon l'une quelconque des revendications 17 à 20, dans laquelle ladite condition est choisie dans le groupe constitué par une vaginose bactérienne, un microbiote vaginal intermédiaire, et des infections par des levures.

22. Composition destinée à être utilisée selon l'une quelconque des revendications 1 à 16, destinée à être utilisée dans la prophylaxie et/ou le traitement d'une condition affectant le tractus urogénital et causée par C. albicans.

23. Composition destinée à être utilisée selon la revendication 22, ladite composition ayant une activité antimicrobienne.

24. Composition destinée à être utilisée selon la revendication 22 ou 23, ladite condition impliquant un pH élevé et/ou un microbiote perturbé dans le tractus urogénital.

25. Composition destinée à être utilisée selon la revendication 24, dans laquelle ledit microbiote perturbé est **caractérisé par** des niveaux anormalement élevés de bactéries associées aux BV (vaginoses bactériennes) (bactéries anaérobies gram négatif, cocci anaérobies gram positif, staphylocoques, streptocoques, E. coli) et/ou de Candida, en combinaison avec des niveaux anormalement bas de lactobacilles.

26. Composition destinée à être utilisée selon l'une quelconque des revendications 22 à 25, dans laquelle ladite condition est choisie dans le groupe constitué par une vaginose bactérienne, un microbiote vaginal intermédiaire, et des infections par des levures.
